# EUROPÄISCHE PATENTSCHRIFT

(11) **EP 1 853 203 B1**
(45) Veröffentlichungstag und Bekanntmachung des Hinweises auf die Patenterteilung: **01.07.2009**
(21) Anmeldenummer: 06722497.2
(22) Anmeldetag: 16.02.2006
(51) Int. Cl.: A61F 2/82

(54) **STENTPROTHESE FÜR DIE GEFÄSSCHIRURGIE INSBESONDERE IM BEREICH DES AORTENBOGENS**
STENT PROSTHESIS FOR VASCULAR SURGERY, PARTICULARLY IN THE AREA OF THE AORTIC ARCH
ENDOPROTHESE POUR CHIRURGIE VASCULAIRE, EN PARTICULIER DANS LA ZONE DE LA CROSSE AORTIQUE

(30) Priorität: 17.02.2005 DE 102005007341
(43) Veröffentlichungstag der Anmeldung: 14.11.2007
(73) Patentinhaber: Kasprzak, Piotr, 93051 Regensburg (DE)
(72) Erfinder: Kasprzak, Piotr, 93051 Regensburg (DE)
(74) Vertreter: Viering, Jentschura & Partner
(86) Internationale Anmeldenummer: PCT/DE2006/000285
(87) Internationale Veröffentlichungsnummer: WO 2006/086969

(56) Entgegenhaltungen:
- EP-A- 0 894 481
- US-A- 6 099 548
- US-A1- 2003 135 257

## Beschreibung

Die Erfindung betrifft eine Stentprothese für die Gefäßchirurgie, insbesondere eine Stentprothese zur Überbrückung einer Gefäßerweiterung im Bereich des Aortenbogens, d.h. beispielsweise direkt oder nahe am oder auch in einem gewissen Abstand vom Aortenbogen.

Seit vielen Jahren werden in der Gefäßchirurgie, Radiologie und Herzchirurgie Stents, auch als Stentprothesen oder Stentimplantate bezeichnet, zur Ausschaltung von Aortenaneurysmen angewandt. Bei einer derartigen Stentprothese handelt es sich um einen rohrförmigen Stentkörper mit einem Umfangsmantel aus im allgemeinen flüssigkeitsdichtem Material, insbesondere Kunststoffmaterial, der flexibel biegbar und radial expandierbar ist. Dieser Stentkörper wird im radial zusammengefalteten Zustand in einem Einführrohr beispielsweise eines Katheters durch die Schlagader hindurch bis zur Anwendungsstelle eingeführt und dort vom Katheter freigegeben, so daß der Stentkörper expandiert wird oder sich entfaltet und sich wenigstens mit seinem beim Einführen vorderen Endabschnitt und hinteren Endabschnitt insbesondere vor und hinter einer Gefäßerweiterung dicht an die Gefäßwand anlegt. Im allgemeinen ist in der Umfangswand des Stentkörpers ein Drahtgittermaterial oder Drahtgeflecht vorgesehen, welches durch Eigenelastizität radial expandiert, wenn der Stentkörper aus dem Einführrohr des Katheters zur Freigabe des Stents herausgeschoben oder das Einführrohr zur Freigabe des Stents zurückgezogen wird, oder wenn der Stentkörper - z.B. mit Hilfe eines Ballonkatheters - expandiert wird.

Der zusammengefaltete Stent wird beispielsweise durch einen Hautschnitt in der Leiste über die darunter liegende Schlagader bis in die Hauptschlagader (Aorta) eingeführt und dort so entfaltet, daß die Gefäßerweiterung oder Aussackung komplett überbrückt oder ausgeschaltet ist. Dazu ist es erforderlich, daß der Stent vor und nach der Aussackung sicher verankert werden kann und so abdichtet, daß keine direkte Bluteinströmung in die Aussackung mehr möglich ist. Dies geschieht im fließenden Blut und unter Röntgendurchleuchtung, wodurch das genaue Einpassen kompliziert ist.

Eine besondere Schwierigkeit ergibt sich im Bereich der Brustkorbhauptschlagader (thorakale Aorta) vor allem in solchen Fällen, in welchen die armversorgende Schlagader oder die Halsschlagader zum Teil oder komplett an ihrem Abgang aus der Hauptschlagader überdeckt wird. Die komplett verschlossenen wichtigen Seitenäste müssen unter Umständen unter Mithilfe von Bypassverfahren mitversorgt werden. Voraussetzung ist allerdings, dass mindestens eine an dem Aortenbogen abgehende Hauptseitenader (Truncus brachio-cephalicus) offen bleibt. Noch schwieriger wird es, eine seitliche Öffnung in der Stentprothese, welche genauestens plaziert werden muß, so vor den Abgang der Seitenader (z.B. Truncus brachio-cephalicus) vorzubringen, daß der Blutfluß in der entsprechenden Schlagader gewährleistet wird. Ein Blockieren dieser Ader würde schwerste Komplikationen verursachen. Das genaue Plazieren des Stents ist durch den bogenförmigen Verlauf der Brustkorbhauptschlagader am Aortenbogen besonders erschwert.

Aus der EP 0 894 481 A2 ist es bekannt, entlang des beispielsweise geflochtenen Außenmantels des Stentkörpers einen langen Markerfaden aus oder mit unter Röntgenstrahlen erkennbarem Material derart anzubringen, dass der Ort und die Position des Stentkörpers während seines Einbringens und Plazierens in der Schlagader anhand des Markerfadens in der Röntgenaufnahme erkannt werden können. Dieser Markerfaden, dessen proximales Fadenende beispielsweise in dem Einbringkatheter fixiert wird, ist an dem Stentkörper derart befestigt, dass er nach der Positionierung des Stentkörpers aus dem Stentkörper herausgezogen werden kann. Es können auch fixiert befestigte, beispielweise verknotete Markerfäden vorgesehen sein, die permanent am Stentkörper verbleiben und sich nicht signifikant vom Stentkörper wegerstrecken.

Als nächstliegender Stand der Technik wird die US 2003/0135257 A1 angesehen, welche die Merkmale des Oberbegrifs des Anspruchs 1 zeigt.

Weiter ist es aus US 2003/0135257 A1 bekannt, die beiden Endbereiche des Stentkörpers, die mit durch Eigenelastizität radial expandierbaren Drahtgittern versehen sind, mit langen Fäden zu umschlingen, die durch eine Reihe von Halteösen an der Außenseite des Stentkörpers hindurchlaufen und an einer der Ösen mit einem derartigen Knoten befestigt sind, daß sich der Knoten durch Ziehen an dem betreffenden Faden löst, wenn sich der Stentköper am Anbringungsort entfalten soll. Ferner ist es aus dieser Druckschrift bekannt, Halteösen für reißfeste lange Fäden an der Außenseite von Verschlußkappen des Stentkörpers zu fixieren; von denen seitliche Öffnungen des Stentkörpers überdeckt werden und die mittels der Fäden, die durch die Ösen hindurchgefädelt sind, am Anbringungsort des Stentkörpers in Seitenadern hinein ausgewölbt werden können, um diese zu verschließen. Danach werden die Fäden durch Ziehen an ihrem einen Fadenende wieder aus den Halteösen herausgezogen. Gemäß US 6 099 548 A kann am vorderen Ende des Stentkörpers eine Reihe von Löchern ausgebildet sein, durch welche lange Fäden als Hilfe zur Positionierung des Stentkörpers hindurchgeführt sein können, die nach der Entfaltung des Stentkörpers wieder herausgezogen werden.

Durch die Erfindung wird eine Stentprothese geschaffen, welche mit einer Plazierungs- und/oder Fixierungshilfe ausgestattet ist.

Gemäß der in den Patentansprüchen beschriebenen Erfindung ist/sind an einer jeweiligen Befestigungsstelle, die an der Außenfläche des Stentkörpers an oder vorzugsweise im Abstand von dem vorderen, (beim Einführen vorlaufenden) Ende des Stentkörpers gelegen ist, mindestens ein langer Faden, gegebenenfalls zwei oder mehr lange Fäden, an der Umfangswand des Stentkörpers mit ihrem einen Fadenende fixiert befestigt. Das Fadenmaterial oder darin enthaltene Materialbestandteile oder eine Umhüllungs des Fadens ist/sind unter Röntgenstrahlung sichtbar. Die jeweilige Befestigungsstelle kann insbesondere in der vorderen Hölfte der Länge des Stentkörpers, beispielsweise im vorderen Drittel oder Viertel der Länge des Stentkörpers gelegen sein. Die Fadenlänge des langen Fadens, der ausgehend von der Befestigungsstelle außerhalb des Stentkörpers freiliegt, ist mit mindestens zehn Zentimetern, bevorzugt mehreren Dezimetern, ausreichend lang, daß der Faden mit seinem freien Fadenende vor der Einführung des Stents beispielsweise durch einen Hautschnitt in der Leiste in den Blutstrom der Hauptschlagader eingebracht werden kann und durch eine Hilfspunktion der Hauptschlagader vom Hals aus, oder beispielsweise durch eine Hilfspunktion eines Seitenastes der Schlagader von einer anderen Körperstelle aus, unter Zuhilfenahme eines speziellen Katheters oder Geräts (z.B. mit Hilfe eines sogenannten Lassokatheters) in der Schlagader eingefangen und aus der Schlagader durch die Punktionsstelle hindurch herausgezogen werden kann.

Dadurch wird erreicht, daß der Stent, welcher auf den Faden folgend in den Blutstrom in der Schlagader eingeschleust wird, mithilfe des Fadens an dem vorgegebenen Anbringungsort der Aorteninnenwand genau ausgerichtet und plaziert werden kann, wozu die jeweilige Punktionsstelle, gegebenenfalls in Anpassung an den Ort einer im Mantel des Stentkörpers ausgebildeten seitlichen Stentöffnung sowie an die Stelle, an welcher der Faden am Stentkörper befestigt ist, vor der Operation bestimmt wird. Dies gilt auch für Stents mit mehr als einer vorgefertigten seitlichen Stentöffnung. Außerdem kann der Stent, der beispielsweise mit seinem vorderen und seinem hinteren Stentende an der Gefäßwand fixiert wird, mit Hilfe des Fadens zusätzlich noch an einer dritten Stelle fixiert werden. Hierzu kann der Faden oder können die Fäden so an der Gefäßwand verankert werden, daß diese dritte Fixation dauerhaft ist.

Der lange Faden oder die langen Fäden sind daher erfindungsgemäß für die Plazierung und/oder Fixierung des Stentkörpers hinreichend zugfest ausgebildet und an dem Stentkörper derart zugfest fixiert befestigt, dass sie sich bei der Manipulation des Stentkörpers nicht von diesem lösen können.

Anstelle eines einzigen langen Fadens, der an der Befestigungsstelle an der Außenfläche des Stentkörpers fixiert ist, können an diesem auch mehrere lange Fäden erfindungsgemäßer Art, insbesondere zwei Fäden an derselben Stelle der Außenfläche, die hier auch als Doppelfaden bezeichnet sind, oder an unterschiedlichen Stellen der Außenfläche fixiert befestigt sein. Beispielsweise können zwei Fäden, von denen einer oder beide als Einzelfaden oder Doppelfaden ausgebildet sein kann/können, an Befestigungsstellen, die an der Außenfläche des Stentkörpers im Abstand voneinander gelegen sind, fixiert befestigt sein. Hierdurch kann mit dem ersten und/oder dem zweiten Faden die Plazierung des Stentkörpers korrigiert werden und die Fixierung des Stentkörpers kann mit Hilfe des ersten und/oder des zweiten Fadens erfolgen.

Die jeweilige Befestigungsstelle kann in Anpassung an die Anatomie des Patienten vor der Operation für eine entsprechende Herrichtung des Stents durch den Hersteller vorgegeben werden. Wenn mehrere erfindungsgemäße Fäden an unterschiedlichen typischen Stellen der Außenfläche des Stents befestigt sind, können gegebenenfalls die nicht zu verwendenden Fäden unmittelbar vor der Operation abgeschnitten werden.

Wenn in dem Stentkörper eine seitliche Öffnung zur Freihaltung des Abgangs einer Seitenader ausgebildet ist, liegt die Befestigungsstelle des Fadens oder insbesondere Doppelfadens vorzugsweise im Abstand von der Öffnung an deren von dem vorderen Ende des Stentkörpers abgewandten Seite. Dieser Abstand kann in Anpassung an die gewählte Punktionsstelle beispielsweise 1 bis 3 cm oder mehr betragen. Bevorzugt liegt außerdem die Befestigungsstelle des Einzel- oder Doppelfadens auf oder nahe an derselben Mantellinie, auf welcher der größte Durchmesser der Öffnung liegt. Auch hierbei können die Orte der seitlichen Öffnung und der Befestigungsstelle des Fadens relativ zueinander vor der Operation vorgegeben werden. Zusätzlich kann ein erfindungsgemäßer langer Faden an der dem vorderen Ende des Stentkörpers zugewandten Seite der Öffnung befestigt sein. Der Faden kann auch an einer anderen Befestigungsstelle, die an der Außenfläche des Stentkörpers gelegen ist, zwischen dem vorderen, d.h. dem distalen Ende des Stentkörpers und dem hinteren, d.h. dem proximalen Ende des Stentkörpers fixiert befestigt sein. Im gegebenen Fall kann auch der oder ein erfindungsgemäß langer Faden an einer Befestigungsstelle befestigt sein, die unmittelbar an dem vorderen Ende oder dem hinteren Ende des Stentköpers gelegen ist.

Eine erfindungsgemäße Stentprothese kann auch in ihrem Stentkörper eine vordere oder hinter seitliche Öffnung aufweisen, welche nach außen hin in einen schlauchförmigen Ärmelabschnitt mündet, der an dem Umfangsmantel des Stentkörpers rings der seitlichen Öffnung dicht befestigt ist. Ein solcher Ärmelabschnitt, von dem die seitliche Öffnung nach außen hin verlängert wird, kann dazu dienen, dass er in eine Seitenader hinein, die von der seitlichen Öffnung des Stentkörpers freigehalten werden soll, ausgestreckt wird. Bei einer solchen Ausführungsform kann wenigstens ein erfindungsgemäßer langer Faden an einer jeweiligen Befestigungsstelle, die an dem freien Rand oder der Außenseite des Ärmelabschnitts gelegen ist, fixiert befestigt sein, so dass der Aermelabschnitt mit Hilfe dieses Fadens in die Seitenader hineingezogen werden und dort im gegebenen Fall an der Gefäßwand fixiert werden kann. In dieser Weise können auch mehrere lange Fäden an dem Ärmelabschnitt befestigt sein.

Bei dieser Ausführungsform kann es genügen, den erfindungsgemäßen langen Faden oder die erfindungsgemäßen langen Fäden nur an dem Ärmelabschnitt vorzusehen. Auch hierdurch läßt sich der Stentkörper im gegebenen Fall bereits insgesamt an seinem Anbringungsort in der Schlagader mittel des Fadens oder der Fäden durch Vermittlung des Ärmelabschnittes plazieren und/oder fixieren. Bei dieser Ausführungsform kann aber auch mindestens ein zusätzlicher langer Faden an dem Umfangsmantel des Stentkörpers befestigt sein.

Die erfindungsgemäße Ausstattung des Stentkörpers mit einem oder mehreren Fäden an der der seitlichen Öffnung abgewandten Seite ist besonders vorteilhaft, weil dadurch mit Hilfe des Fadens eine genaue Plazierung der seitlichen Öffnung ermöglicht ist, was mit Hilfe eines zusätzlichern Fadens an der vorderen Seite der Öffnung noch begünstigt sein kann.

Bei Bedarf kann zur Kontrolle der korrekten Lage des Stents nach Teilentfaltung und vor endgültiger Freisetzung eine gegen die Öffnung entlang des Umfangs des Stentkörpers versetzte zusätzliche seitliche Öffnung im Mantel des Stentkörpers ausgespart sein, die gegenüber der ersten Öffnung auch zum hinteren Ende des Stentkörpers axial versetzt sein kann. Durch eine solche Öffnung kann ein Kontrollkatheter von außen nach innen (in den Stentkörper) und dann wieder von innen nach außen durch die erste Öffnung hindurch in die zu versorgende Seitenader z.B. den Truncus brachio-cephalicus zur Überprüfung eingebracht werden. Gegebenenfalls kann durch die erste Öffnung hindurch eine zusätzliche Fixation der Stentöffnung in der Seitenader mit einem zusätzlichen Stent erfolgen. Die zusätzliche seitliche Öffnung wird dann am Ende der Operation verschlossen, z.B. mit einem zusätzlichen Stent.

Zur Einführung eines erfindungsgemäßen Stents kann ein Katheter verwendet werden, bei welchem im freien Endbereich des Einführrohrs wenigstens ein dessen Rohrmantel durchdringender und bis zu dessen Stirnende durchgehender, vorzugsweise axialer Schlitz ausgebildet ist, durch welchen der jeweilige Faden vor der Ausbringung des zusammengefalteten Stents aus dem Katheder herausgeführt werden kann. Der Schlitz ermöglicht, daß der Stent mittels des eingebrachten Fadens noch vor dessen Austreten aus dem freien Endabschnitt des Einführrohrs des Katheters und daher noch vor dem Entfalten des Stents direkt an die gewünschte Stelle plaziert und an dieser festgehalten werden kann, ohne daß es während der Freisetzung des Stents zu einer Verschiebung desselben kommen kann.

Die Erfindung wird mit Hilfe von bevorzugten Ausführungsformen erläutert, die aus der Zeichnung ersichtlich sind. In der Zeichnung zeigt:
- Fig. 1.: eine Stentprothese mit Öffnung und zwei Fäden am Stentkörper;
- Fig. 2 - 4.: Unterschiedliche Möglichkeiten der Fadenfixation am Stent;
- Fig. 5 - 8.: Beispiele für unterschiedliche Fadenbefestigungen;
- Fig. 9.: Ein Beispiel für eine erfindungsgemäße Stentprothese mit einem seitlichen Ärmelabschnitt;
- Fig. 10: Ein Beispiel für einen Einführungskatheter mit Schlitz und äußerlicher Hülle;
- Fig. 11.: einen Stent mit einer zweiten Öffnung;

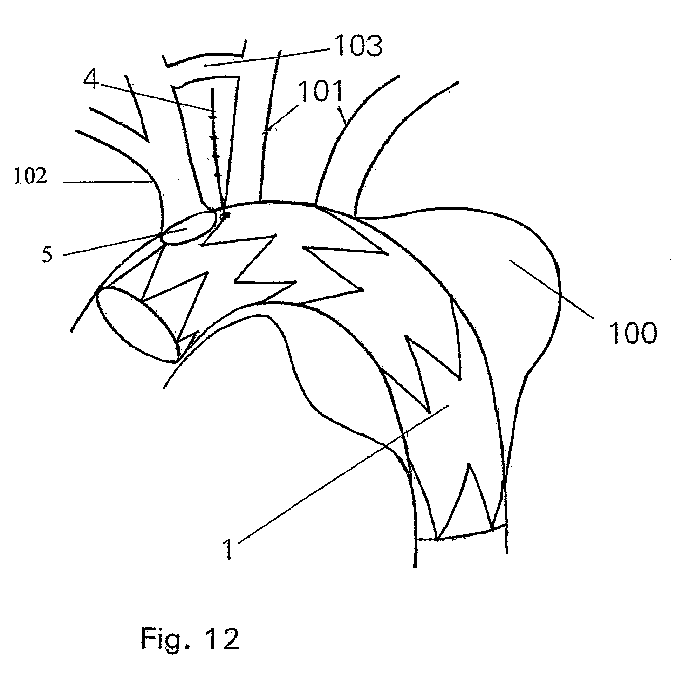

- Fig. 12.: Ein Anwendungsbeispiel für die Stenteinbringung in die Aorta.

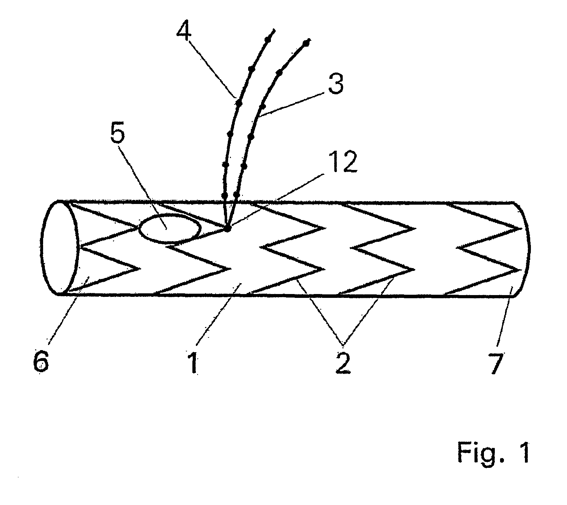

Wie aus Fig. 1 ersichtlich, ist am rohrförmigen Stentkörper 1, in dem an oder in dessen Umfangsmantel, der mit Ausnahme an vorgesehenen Öffnungen für Blut flüssigkeitsdicht ist, ein Drahtgitter oder Drahtgewebe oder Drahtgeflecht 2 angeordnet ist, im vorderen Viertel der Länge des Stentkörpers im Abstand von dessen vorderem Ende 6 eine seitliche Öffnung 5 ausgebildet. Im geringen Abstand hinter der Öffnung 5 zu dem hinteren Ende 7 des Stentkörpers hin versetzt sind zwei erfindungsgemäße lange Fäden 3, 4 als Doppelfaden befestigt. In die Fäden ist ein Metalldraht oder sind Metallpartikel oder dergleichen einbezogen, so daß die Fäden während der Operation in Röntgenstrahlung erkennbar sind. Der oder die Fäden können aber auch mit einer Umhüllung aus einem in Röntgenstrahlung erkennbaren Material versehen sein, so dass das Fadenmaterial als solches in Röntgenstrahlung nicht erkennbar sein muss.

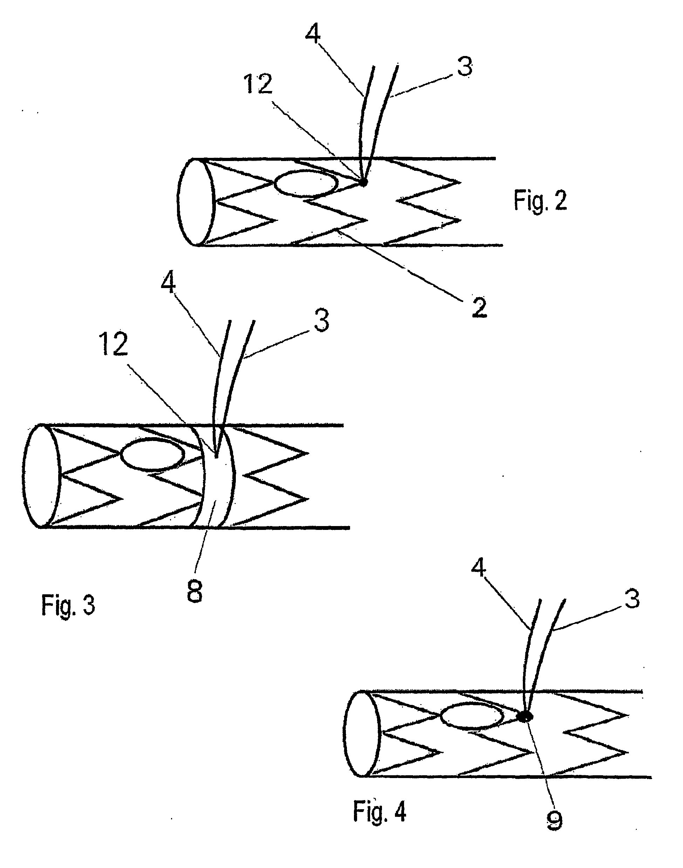

Die Fäden 3, 4 können an ihrer Befestigungsstelle 12, die an der Außenfläche des Stentkörpers gelegen ist, z.B. am Metallgitter 2 (Fig. 2), an einem rings des Stentkörpers an diesem angebrachten Verstärkungsband 8 (Fig. 3), an einem auf die Umfangsfläche des Stentkörpers aufgesetzten Flicken 9 (Fig. 4) oder in sonstiger Weise derart am Stentkörper befestigt sein, daß sie bei der Einbringung und Plazierung des Stents und auch nach der Opersation nicht ausreißen können.

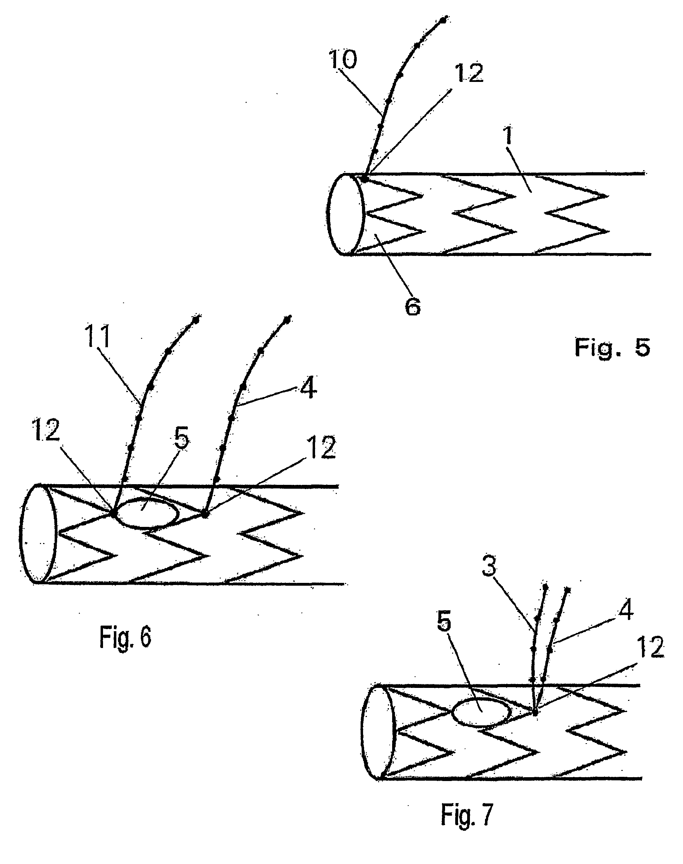

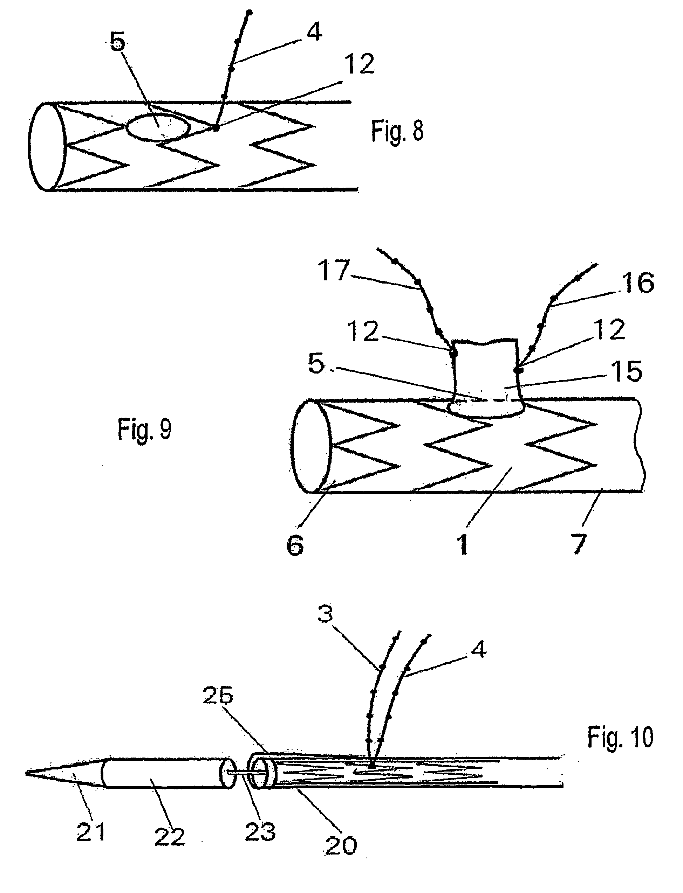

Aus den Figuren 5 bis 8 sind weitere Ausführungsbeispiele der Erfindung ersichtlich als Stent ohne Öffnung, jedoch mit einem erfindungsgemäßen Faden 10 am vorderen Ende 6 des Stentkörpers 4 (Fig. 5), als Stent mit Öffnung 5 und einem erfindungsgemäßen Faden 11 vor der Öffnung 5 und einem erfindungsgemäßen Faden 4 hinter der Öffnung 5 (Fig. 6), als Stent mit zwei Fäden 3, 4 als Doppelfaden hinter der Öffnung 5 wie gemäß Fig. 1, oder als Stent mit einem Einzelfaden 4 hinter der Öffnung 5 (Fig. 8).

Aus Fig. 9 ist ein Beispiel eines erfindungsgemäßen Stentkörpers 1 ersichtlich, der rings einer seitlichen Öffnung 5 einen Ärmelabschnitt 15 aufweist, der in einen Seitenast der Schlagader hinein ausgedehnt werden soll. Hierzu sind an diametral gegenüberliegenden Befestigungsstellen 12 an der Außenseite des Ärmelabschnitts zwei erfindungsgemäße lange Fäden 16, 17 befestigt. Wenigstens ein zusätzlicher langer Faden kann an einer geeigneten Befestigungsstelle an der Außenumfangsfläche des Stentköpers vorgesehen sein (nicht gezeigt).

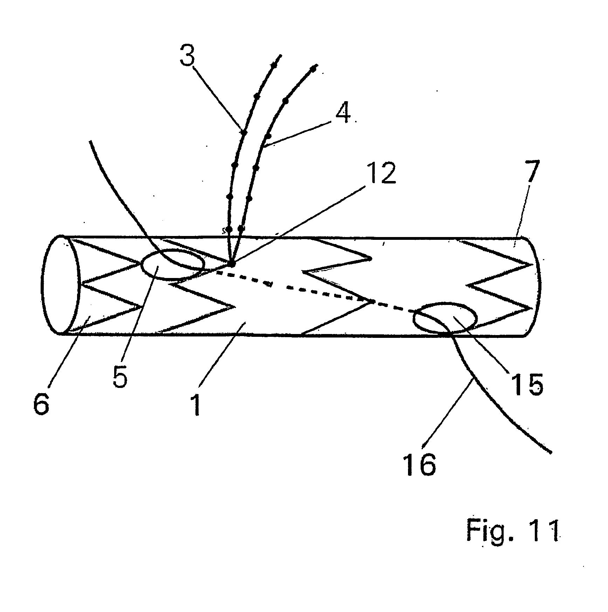

Wie aus Fig. 11 ersichtlich, kann beispielsweise axial im Abstand von der Öffnung 5 zum hinteren Ende des Stentkörpers 1 hin und gegen die Öffnung 5 im Beispiel um etwa 45. Winkelgrad oder mehr am Umfang des Stentkörpers versetzt eine zusätzliche seitliche Öffnung 15 im Mantel des Stentkörpers 1 ausgebildet sein. Die zusätzliche Öffnung 15 bietet eine Kontrollmöglichkeit für die Einführung eines Kontrollkatheters 16 durch die Öffnungen 15 und 5 und gegebenenfalls für die Einbringung eines zusätzlichen Stents (nicht gezeigt) durch ein Einführkatheter 16 zur Fixierung der Öffnung 5 in der sich anschließenden Seitenader.

Aus Fig. 9 ist das distale Ende eines Katheter-Einführrohrs 20 mit darin eingefaltet angeordnetem erfindungsgemäßen Stent (nicht gezeigt) ersichtlich. Im freien Endbereich des Einführrohrs 20 ist wenigstens ein dessen Rohrmantel durchdringender und bis zu dessen Stirnende durchgehender, axialer Schlitz 25 ausgebildet, durch welchen der jeweilige Faden 3, 4 vor der Ausbringung des zusammengefalteten Stents aus dem Katheder herausgeführt werden kann. Die an dem Katheterdraht 23 befestigte Katheterspitze 21 weist eine davon proximal abstehende Hülle 22 auf, die über das freie Ende des Einführrohres 20 bis zu den seitlich herausgeführten Fäden 3, 4 geschoben werden kann, um den geschlitzten Katheterabschnitt und damit den Stent darin zusammenzuhalten, und die vor dem Zurückziehen des Einführrohres 20 von dem Endbereich des Einführrohres mit Hilfe des Katheterdrahtes 23 weggeschoben werden kann.

Aus Fig. 11 ist ein Anwendungsbeispiel für die Einbringung eines erfindungsgemäßen Stentkörpers 1 in den Aortenbogen zur Überbrückung einer Aussackung 100 skizziert. Der Stentkörper 1 ist hinreichend flexibel biegbar, daß er der Krümmung des Aortenbogens folgen kann. Von der seitlichen Öffnung 5 in dem Stentkörper 1 wird der abgehende Truncus brachio-cephalicus 102 freigehalten, wohingegen die anderen beiden abgehenden Schlagadern 101 von dem Stentkörper blockiert werden; durch einen Bypass 103 ist für deren Blutversorgung gesorgt. Mithilfe des erfindungsgemäß an dem Stentkörper 1 befestigten langen Fadens 4 kann der Stentkörper 1 während seiner Einbringung plaziert und anschließend in seiner Lage fixiert werden.

## Patentansprüche

1. Stentprothese für die Gefäßchirurgie zur Einbringung in eine Schlagader, insbesondere zur Überbrückung einer Gefäßerweiterung im Bereich des Aortenbogens, mit einem rohrförmigen flexiblen und radial expandierbaren Stentkörper (1) mit einer Außenfläche und einem für Blut flüssigkeitsdichten Umfangsmantel mit einem vorderen Ende (6) und einem hinteren Ende (7), und wenigstens einem zugfesten langen Faden (3, 4, 10, 11, 16, 17) , der unter Röntgenstrahlung erkennbar ist und eine freie Fadenlänge von mehreren Dezimetern oder von wenigstens zehn Zentimetern hat, **dadurch gekennzeichnet, daß** der lange Faden (3, 4, 10, 11, 16, 17) an einer jeweiligen Befestigungsstelle (12), die an der Außenfläche des Stentkörpers im Abstand von dem vorderen Ende (6) und dem hinteren Ende des Umfangsmantels gelegen ist, mit seinem einen Fadenende zugfest fixiert befestigt ist und von seiner Befestigungsstelle (12) aus außerhalb des Stentkörpers freiliegt, so daß der lange Faden mit seinem freien Fadenende vor dem Stentkörper in die Schlagader eingeschleust und in der Schlagader eingefangen werden kann und der eingeschleuste Stentkörper mittels des Fadens in seiner Lage in der Schlagader plaziert und anschließend mittels des Fadens an der Gefäßwand fixiert werden kann.

2. Stentprothese nach Anspruch 1, wobei der lange Faden (3, 4) ein Doppelfaden ist.

3. Stentprothese nach Anspruch 1 oder 2, wobei in dem Umfangsmantel des Stentkörpers (1) eine seitliche Öffnung (5) ausgebildet ist und die Befestigungsstelle (12) des langen Fadens (3, 4) an dem Umfangsmantel in geringem Abstand von der seitlichen Öffnung angeordnet ist.

4. Stentprothese nach Anspruch 3, wobei die Befestigungsstelle (12) des langen Fadens (3, 4) an dem Umfangsmantel des Stentkörpers an der dem vorderen Ende (6) des Stentkörpers (1) abgewandten Seite der seitlichen Öffnung (5) angeordnet ist.

5. Stentprothese nach Anspruch 4, wobei an einer Befestigungsstelle (12) an der dem vorderen Ende (6) des Umfangsmantels des Stentkörpers (1) zugewandten Seite der seitlichen Öffnung (5) ein zusätzlicher langer Faden (11) an dem Umfangsmantel fixiert befestigt ist.

6. Stentprothese nach einem der Ansprüche 1 bis 5, wobei in dem Umfangsmantel des Stentkörpers (1) eine seitliche Öffnung (5) ausgebildet ist und der Stentkörper einen Ärmelabschnitt (15) aufweist, von dem die seitliche Öffnung (5) außerhalb des Umfangsmantels verlängert wird, wobei die Befestigungsstelle (12) des langen Fadens (16, 17) an dem Ärmelabschnitt angeordnet ist.

7. Stentprothese nach einem der Ansprüche 3 bis 6, wobei im Umfangsmantel des Stentkörpers (1) in dessen Umfangsrichtung gegen die Öffnung (5) versetzt eine zweite Öffnung (15) ausgebildet ist, die bevorzugt gegenüber der ersten Öffnung (5) zum hinteren Ende (7) des Stentkörpers hin axial versetzt ist.

## Claims

1. Stent prosthesis for vascular surgery for inserting into an artery, particularly for bridging a vasodilation in the area of the aortic arch, comprising a tubular flexible and radially expandable stent body (1) including an outer surface and a peripheral sheathing impermeable to blood comprising a front end (6) and a rear end (7), and at least one tensile-proof long thread (3, 4, 10, 11, 16, 17) being visible under x-radiation and having a free thread length of several decimetres or of at least ten centimetres, **characterised in that** the long thread (3, 4, 10, 11, 16, 17) is attached with its one thread end in a fixed and tensile-proof manner to a respective point of attachment (12) situated at the outer surface of the stent body at a distance from the front end (6) and the rear end of the peripheral sheathing, and is exposed starting from its point of attachment (12) outside the stent body, so that the long thread may be introduced with its free thread end in advance of the stent body into the artery and may be caught in the artery, and so that the introduced stent body may be placed in its position in the artery by means of the thread and may subsequently be fixed to the vascular wall by means of the thread.

2. Stent prosthesis according to claim 1, wherein the long thread (3, 4) is a double thread.

3. Stent prosthesis according to claim 1 or 2, wherein a lateral opening (5) is provided in the peripheral sheathing of the stent body (1), and the point of attachment (12) of the long thread (3, 4) is arranged on the peripheral sheathing at a short distance from the lateral opening.

4. Stent prosthesis according to claim 3, wherein the point of attachment (12) of the long thread (3, 4) is arranged on the peripheral sheathing of the stent body on the side of the lateral opening (5) averted from the front end (6) of the stent body (1).

5. Stent prosthesis according to claim 4, wherein an additional long thread (11) is fixedly attached to the peripheral sheathing at a point of attachment (12) on the side of the lateral opening (5) facing the front end (6) of the peripheral sheathing of the stent body (1).

6. Stent prosthesis according to any of claims 1 to 5, wherein a lateral opening (5) is provided in the peripheral sheathing of the stent body (1), and the stent body comprises a sleeve portion (15) by which the lateral opening (5) is extended outside the peripheral sheathing, the point of attachment (12) of the long thread (16, 17) being disposed on the sleeve portion.

7. Stent prosthesis according to any of claims 3 to 6, wherein a second opening (15) is provided in the peripheral sheathing of the stent body (1) in its circumferential direction offset against the opening (5), which is preferably offset axially against the first opening (5) towards the rear end (7) of the stent body.

## Revendications

1. Prothèse de stent pour la chirurgie vasculaire pour introduction dans une artère, en particulier pour ponter une vasodilatation dans la région de l'arc aortique, comportant un corps de stent (1) tubulaire, flexible, et radialement dilatable comprenant une surface extérieure et une enveloppe circonférentielle imperméable au sang avec une extrémité antérieure (6) et une extrémité postérieure (7), et au moins un fil long et résistant à la traction (3, 4, 10, 11, 16, 17) qui est visible sous rayons X et possède une longueur de fil libre de plusieurs décimètres ou d'au moins dix centimètres, **caractérisée en ce que** le long fil (3, 4, 10, 11, 16, 17) est attaché par la une extrémité de fil de façon fixe et résistante à la traction à un point d'attachement (12) respectif qui est situé sur la surface extérieure du corps de stent à distance de l'extrémité antérieure (6) et de l'extrémité postérieure de l'enveloppe circonférentielle et est exposé à partir de sa position d'attachement (12) hors du corps de stent, de façon que le fil long puisse être introduit dans l'artère par son extrémité de fil libre avant le corps du stent et puisse être capté à l'intérieur de l'artère, et que le corps de stent introduit puisse être placé en position dans l'artère au moyen du fil et puisse alors être fixé à la paroi vasculaire au moyen du fil.

2. Prothèse de stent selon la revendication 1, dans laquelle le fil long (3, 4) est un fil double.

3. Prothèse de stent selon la revendication 1 ou 2, dans laquelle une ouverture latérale (5) est formée dans l'enveloppe circonférentielle du corps de stent (1) et dans laquelle la position d'attachement (12) du fil long (3, 4) sur l'enveloppe circonférentielle est située à distance courte de l'ouverture latérale.

4. Prothèse de stent selon la revendication 3, dans laquelle la position d'attachement (12) du fil long (3, 4) sur l'enveloppe circonférentielle du corps de stent est localisée du côté de l'ouverture latérale (5) détourné de l'extrémité antérieure (6) du corps de stent (1).

5. Prothèse de stent selon la revendication 4, dans laquelle un fil long additionnel (11) est attaché à l'enveloppe circonférentielle de manière fixe à une position d'attachement (12) du côté de l'ouverture latérale (5) tourné vers l'extrémité antérieure (6) de l'enveloppe circonférentielle du corps de stent (1).

6. Prothèse de stent selon l'une des revendications 1 à 5, dans laquelle une ouverture latérale (5) est formée dans l'enveloppe circonférentielle du corps de stent (1) et dans laquelle le corps de stent comporte une section de manche (15) par laquelle l'ouverture latérale (5) est prolongée hors de l'enveloppe circonférentielle, la position de l'attachement (12) du fil long (16, 17) étant située sur la section de manche.

7. Prothèse de stent selon l'une des revendications 3 à 6, dans laquelle une deuxième ouverture (15) est formée dans l'enveloppe circonférentielle du corps de stent (1) dans sa direction circonférentielle décalée contre l'ouverture (5), qui est de préférence décalée relativement à la première ouverture (5) de façon axiale vers l'extrémité postérieure (7) du corps du stent.
